# EUROPEAN PATENT SPECIFICATION

(11) **EP 3 733 221 B1**
(45) Date of publication and mention of the grant of the patent: **13.09.2023**
(21) Application number: 18897786.2
(22) Date of filing: 18.12.2018
(51) Int. Cl.: A61L 31/02, A61L 31/08, A61L 31/10, A61L 31/14, A61L 31/16

(54) **IMPLANTABLE DRUG DELIVERY DEVICE**
IMPLANTIERBARE WIRKSTOFFFREISETZUNGSVORRICHTUNG
DISPOSITIF D'ADMINISTRATION DE MÉDICAMENT IMPLANTABLE

(30) Priority: 28.12.2017 CN 201711461899
(43) Date of publication of application: 04.11.2020
(73) Proprietor: Biotyx Medical (Shenzhen) Co., Ltd., Shenzhen, Guangdong 518000 (CN)
(72) Inventor: QIN, Li, Shenzhen, Guangdong 518057 (CN); LIN, Wenjiao, Shenzhen, Guangdong 518057 (CN); LIU, Ziqiang, Shenzhen, Guangdong 518057 (CN); ZHANG, Deyuan, Shenzhen, Guangdong 518057 (CN)
(74) Representative: Prinz & Partner mbB
(86) International application number: PCT/CN2018/121678
(87) International publication number: WO 2019/128767

(56) References cited:
- WO-A1-2017/117923
- WO-A1-2017/117924
- CN-A- 101 161 299
- CN-A- 101 185 779
- CN-A- 102 772 831
- CN-A- 104 857 570
- CN-A- 104 888 282
- CN-A- 106 955 374
- US-A1- 2005 267 560
- Yufeng ZHENG; Hongtao YANG: "Research Progress in Biodegradable Metals for Stent Application", Acta Metallurgica Sinica, vol. 53, no. 10, 31 October 2017 (2017-10-31), pages 1227-1237, XP055718484, DOI: 10.11900/0412.1961.2017.00270

## Description

### Field

The embodiments relate to the field of interventional medical devices, and more particularly relates to an implantable drug-loaded device.

### Background

At the present, common luminal stenosis treatment methods are interventional therapies such as percutaneous transluminal angioplasty, which expands a narrowed lumen to a normal size through an implantable device. Therefore, an implanting position would be partially injured. In the critical period of tissue repair, smooth muscle cells are proliferated most seriously. After the tissue of the lumen has passed through the critical period of repair, the smooth muscle proliferation gradually slows down, and is stabilized. Therefore, an active drug can be loaded on the surface of the implantable device to effectively inhibit the cell proliferation at a lesion, keep the lumen unobstructed, and achieve a therapeutic effect. That is, after the implantable drug-loaded device is implanted in a human body, the active drug is released around the device, and is absorbed and metabolized by the tissue. When the active drug accumulated in the tissue reaches a certain threshold value, the active drug can exert its pesticide effect and play a role in inhibiting the cell proliferation. When the device carries the same amount of a drug, if the release rate of the active drug is lower, the content of the active drug accumulated in the tissue is smaller, resulting in a failure of inhibiting the cell proliferation around the device; and if the release rate of the active drug is higher, the content of the active drug accumulated in the tissue is larger, which easily inhibits the endothelialization of the lumen of an implanted section of the device and even causes necrosis of normal histocytes. When the release rate of the active drug is constant, if fewer active drugs are loaded, the content of the active drugs accumulated in the tissue is smaller, which may lead to a failure of effectively inhibiting the cell proliferation around the device; and if more active drugs are loaded, the content of the active drugs accumulated in the tissue is larger, which may also lead to the inhibition to the endothelialization of the lumen of the implanted section of the device and even kill normal cells. At the same time, longer release time of the active drug may cause higher toxicity to the tissue, so that an appropriate drug release rate should be selected. On the basis of creating an effective tissue active drug concentration, the lower the drug loading capacity, the better.

Zinc, as a bio-corrodible material, can be used to prepare a fully absorbable device, and thus has gradually attracted people's attention. Corrosion occurs after implantation in the human body. Zinc corrosion products include metal ions and solid products. Previous studies have shown that the zinc corrosion products released during the zinc corrosion can inhibit the smooth muscle cell proliferation when reaching a certain concentration, thereby deducing that the luminal stenosis rate of the tissue around the device can be reduced. However, if excessive zinc is released during the zinc corrosion, it will kill smooth muscle cells, and even endothelial cells and other normal histocytes, eventually leading to tissue ulceration or necrosis at the implanting position. Documents WO 2017/117924 A1 and WO 2017/117923 A1 disclose an iron based stent comprising a Zn layer on which a coating layer comprising PLA and sirolimus is deposited.

When the implantable drug-loaded device can release both the zinc and a drug that inhibits vascular tissue proliferation, the zinc and the drug will work together on vascular tissues to synergistically achieve an effect of inhibiting vascular neointimal hyperplasia. If not controlled, the effect is easily enhanced to generate toxicity to cause adverse tissue reaction and even tissue necrosis. Therefore, it is necessary to design a reasonable drug release concentration for a zinc-containing drug-loaded device to ensure that after the zinc-containing implantable drug-loaded device is implanted in a human body, the zinc and the drug can synergistically work to achieve the objectives of effectively inhibiting the smooth muscle cell proliferation and maintaining an unobstructed lumen, and there will be no adverse reactions and even necrosis in the tissue.

### Summary

Based on this, it is necessary to provide an implantable drug-loaded device which can enable the concentration of zinc corrosion products in a tissue to be matched with the concentration of an active drug to achieve the objectives of inhibiting smooth muscle cell proliferation and generating no toxic and side effects.

The references to methods of treatment in the summary and detailed description of the invention in this description are to be interpreted as references to the compounds, pharmaceutical compositions and medicaments of the present invention for use in a method for treatment of the human (or animal) body by therapy.

The present invention provides an implantable drug-loaded device as defined in claim 1.

In one embodiment, the zinc-containing matrix is a matrix formed by zinc or a zinc alloy. Or, the zinc-containing matrix includes a body and zinc-containing portions attached to the body, and a material of the zinc-containing portions is pure zinc or a zinc alloy.

In one embodiment, the zinc alloy is an alloy formed by zinc and at least one of iron, magnesium, palladium, rhenium, gold, platinum, tantalum, and strontium; or the zinc alloy is an alloy formed by doping zinc with at least one of carbon, nitrogen, oxygen, sulfur, boron, and silicon.

In one embodiment, when the zinc-containing matrix includes the body, and the zinc-containing portions attached to the body, and the material of the zinc-containing portions is the pure zinc or the zinc alloy, the zinc-containing portions at least partially cover the surface of the body, or the zinc-containing portions fill the inside of the body.

In one embodiment, when the zinc-containing matrix includes the body, and the zinc-containing portions attached to the body, and the material of the zinc-containing portions is the zinc alloy, and the body is a metal body, the zinc-containing portions are formed by reaction between the metal body and pure zinc.

In one embodiment, when the zinc-containing matrix includes the body, and the zinc-containing portions attached to the body, and the material of the zinc-containing portions is the pure zinc or the zinc alloy, the active drug layer does not cover the zinc-containing portions at all, or the active drug layer at least partially covers the zinc-containing portions.

In one embodiment, the active drug layer includes an active drug and a carrier, and the active drug is dispersed in the carrier.

In one embodiment, the active drug is at least one of heparin, colchicine, rapamycin and a derivative thereof, paclitaxel and a derivative thereof, and hirudin and a derivative thereof.

In one embodiment, the carrier is at least one of a degradable polymer, a non-degradable polymer, and a copolymer formed by at least two of monomers that form the degradable polymer and monomers that form the non-degradable polymer. The degradable polymer is at least one of polylactic acid, polyglycolic acid, polycaprolactone, polysuccinate, and poly(β-hydroxybutyrate), and the non-degradable polymer is at least one of polystyrene, polytetrafluoroethylene, polymethyl methacrylate, polycarbonate, and polyethylene terephthalate.

In one embodiment, the body is made of a metal, and the metal is a degradable metal or a non-degradable metal.

In one embodiment, the degradable metal is pure iron, an iron alloy, pure magnesium, or a magnesium alloy.

In one embodiment, the non-degradable metal is 316L stainless steel, a cobalt-chromium alloy, a platinum-chromium alloy, pure titanium or a titanium alloy.

In one embodiment, the body is at least partially made of a polymer. The polymer is at least one of a degradable polymer, a non-degradable polymer, and a copolymer formed by at least two of monomers that form the degradable polymer and monomers that form the non-degradable polymer. The degradable polymer is at least one of polylactic acid, polyglycolic acid, polycaprolactone, polysuccinate, and poly(β-hydroxybutyrate), and the non-degradable polymer is at least one of polystyrene, polytetrafluoroethylene, polymethyl methacrylate, polycarbonate, and polyethylene terephthalate.

In one embodiment, the implantable drug-loaded device is a vascular scaffold, a biliary scaffold, an esophageal scaffold, a urethral scaffold, an airway scaffold, an andrology implant, a gynecological implant, or a vena cava filter.

The above-mentioned implantable drug-loaded device includes the zinc-containing matrix and the active drug layer. The content p of the active drug in the zinc-containing matrix per unit area and the content p of the zinc corroded from the zinc-containing matrix per unit area in the Guaranteed grade acetic acid aqueous solution at the concentration of 8.3 vol.% within unit time are reasonably set, so that the zinc release rate and the content of the active drug are matched. Therefore, after the implantable device is implanted into a human body, the zinc and the drug work synergistically to effectively inhibit smooth muscle cell proliferation, and cannot kill cells and cause necrosis in tissues.

### Brief Description of the Drawings

FIG. 1 is a schematic structural diagram of a zinc-containing matrix of an implantable drug-loaded device according to one embodiment;
FIG. 2 is a schematic structural diagram of the zinc-containing matrix of the implantable drug-loaded device shown in FIG. 1 after thermal treatment;
FIG. 3 is a pathological section diagram of Embodiment 1;
FIG. 4 is a pathological section diagram of a comparative example 1;
FIG. 5 is a pathological section diagram of a comparative example 2.

### Detailed Description of the Embodiments

In order to understand the features, objectives and effects of the embodiments more clearly, the specific embodiments are now described in detail with reference to the accompanying drawings, but the scope of protection of the embodiments is as defined in the claims.

Unless otherwise defined, all technical and scientific terms used herein are the same as meanings of general understandings of those skilled in the art of the embodiments. The terms used in the description of the embodiments herein are merely to describe the specific embodiments, not intended to limit the embodiments.

The zinc-containing matrix is a lumen component or a component of other shapes, such as a component of a vena cava filter structure. The zinc-containing matrix is a matrix formed by pure zinc or a zinc alloy. For example, the zinc-containing matrix is a matrix of a lumen structure formed by pure zinc. For example, the zinc alloy is an alloy formed by zinc and at least one of iron, magnesium, palladium, rhenium, gold, platinum, tantalum, and strontium. Or, the zinc alloy is an alloy formed by doping zinc with at least one of carbon, nitrogen, oxygen, sulfur, boron, and silicon.

In another embodiment, the zinc-containing matrix includes a body, and zinc-containing portions attached to the body.

The body is a lumen component or a component of other shapes. For example, the body may be made of a degradable metal or a non-degradable metal. For example, the degradable metal is pure iron, an iron alloy, pure magnesium, or a magnesium alloy. The non-degradable metal is 316L stainless steel, a cobalt-chromium alloy, a platinum-chromium alloy, pure titanium or a titanium alloy.

Alternatively, the body is at least partially made of a polymer. The polymer is at least one of a degradable polymer, a non-degradable polymer, and a copolymer formed by at least two of monomers that form the degradable polymer and monomers that form the non-degradable polymer. The degradable polymer is at least one of polylactic acid, polyglycolic acid, polycaprolactone, polysuccinate, and poly(β-hydroxybutyrate). The non-degradable polymer is at least one of polystyrene, polytetrafluoroethylene, polymethyl methacrylate, polycarbonate, and polyethylene terephthalate.

It can be understood that the material of the body is not limited to the above-mentioned materials, and any material that provides a certain supporting effect and has good biocompatibility may be used to prepare the body.

A material of the zinc-containing portion is pure zinc or a zinc alloy. For example, the zinc alloy is an alloy formed by zinc and at least one of iron, magnesium, palladium, rhenium, gold, platinum, tantalum, and strontium. Or, the zinc alloy is an alloy formed by doping zinc with at least one of carbon, nitrogen, oxygen, sulfur, boron, and silicon.

In one embodiment, the zinc-containing portions are of a layered structure, which may be a continuous layered structure or a discontinuous layered structure. Whether it is the continuous layered structure or the discontinuous layered structure, the zinc-containing portions may be uniform or non-uniform in thickness. The zinc-containing portions may also be structures which are of a regular or irregular dot shape, block shape, line shape, belt shape and the like, are distributed in a scattered manner on the surface of the body, and are formed of the zinc or the zinc alloy. Furthermore, the zinc-containing portions of the regular or irregular dot shape, block shape, line shape, belt shape and the like may be uniformly or non-uniformly distributed. The zinc-containing portions which are distributed in a scattered manner on the surface of the body and are of the regular or irregular dot shape, block shape, line shape, belt shape and the like may be equal or unequal in thickness. The zinc-containing portions at least partially cover the surface of the body. The surface of the body refers to an outer surface, an inner surface or a side surface of the body. If the body has a radiopaque structure which is filled with a radiopaque marker, an exposed area of the radiopaque marker is also considered as a part of the surface area of the body. The zinc-containing portions at least partially covering the surface of the body means that the zinc-containing portions completely cover the surface of the body or the zinc-containing portions only partially cover the surface of the body. For example, the zinc-containing portions cover only the outer surface, or only the inner surface, or only the side surface. Or, the zinc-containing portions may be disposed on the outer surface, the inner surface, or the side surface of the body at the same time, but the outer surface, the inner surface, or the side surface of the body are not completely covered, respectively.

In other embodiments, the zinc-containing portions fill the inside of the body. For example, grooves or microholes are formed in the body, and the pure zinc or the zinc alloy fills the grooves or are embedded in the microholes to form the zinc-containing portions filling the inside of the body.

It can be understood that the above-mentioned zinc alloy refers to a zinc-based alloy whose zinc content is dominant.

It can be understood that when the zinc-containing matrix includes the body, and the zinc-containing portions attached to the body, and the material of the zinc-containing portions is the pure zinc or the zinc alloy, the material of the body may not be the pure zinc or the zinc-based alloy. The material of the body may contain zinc, but the content of the zinc is small. For example, an iron-based alloy or a magnesium-based alloy may contain a little of zinc. The material of the body may not contain zinc, either, such as a polymer body.

The active drug layer is of a layered structure, which may be a continuous layered structure or a discontinuous layered structure. The active drug layer is disposed on the surface of the zinc-containing matrix. When the zinc-containing matrix is a matrix formed by the pure zinc or the zinc alloy, the active drug layer completely covers the surface of the zinc-containing matrix, or the active drug layer partially covers the surface of the zinc-containing matrix. When the zinc-containing matrix includes the body, and the zinc-containing portions attached to the body, and the active drug layer is disposed on the body, the active drug layer completely covers the zinc-containing portions or the active drug layer at least partially covers the zinc-containing portions.

The active drug layer includes an active drug and a carrier. The active drug is dispersed in the carrier.

The active drug is at least one of heparin, colchicine, rapamycin and a derivative thereof, paclitaxel and a derivative thereof, and hirudin and a derivative thereof.

For example, the carrier is at least one of a degradable polymer, a non-degradable polymer, and a copolymer formed by at least two of monomers that form the degradable polymer and monomers that form the non-degradable polymer. The degradable polymer is at least one of polylactic acid, polyglycolic acid, polycaprolactone, polysuccinate, and poly(β-hydroxybutyrate), and the non-degradable polymer is at least one of polystyrene, polytetrafluoroethylene, polymethyl methacrylate, polycarbonate, and polyethylene terephthalate.

It can be understood that the carrier is not limited to the above-mentioned substances. Any pharmacologically acceptable carrier that has film-forming property and may enable the active drug to be loaded on the zinc-containing matrix may be applied into the foregoing active drug layer.

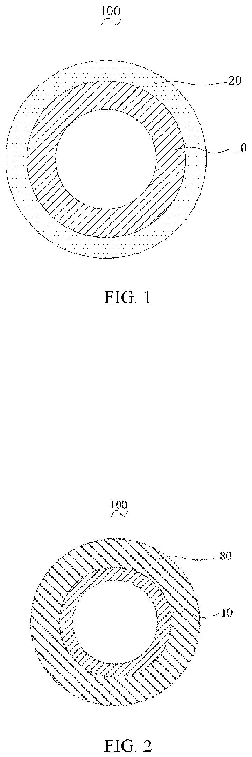

When the zinc-containing matrix includes the body and the zinc-containing portions attached to the body, and the body is a metal body, a pure zinc layer may be firstly formed on the surface of the metal body, and then the pure zinc layer and a partial region of the metal body are alloyed by thermal treatment to form the zinc alloy, i.e., the zinc-containing portions. As shown in FIG. 1, a zinc-containing matrix 100 according to one embodiment includes a metal body 10, and a pure zinc layer 20 covering the outer surface of the metal body 10. The zinc-containing matrix 100 is alloyed. By comparison of FIG. 1 and FIG. 2, after the alloying treatment, the pure zinc layer 20 and a partial region of the metal body 10, which is close to the pure zinc layer 20, are alloyed to form a zinc alloy 30. The partial region of the metal body 10 refers to a region extending from the surface of the metal body 10, which is close to the pure zinc layer 20, in a radial direction toward a direction close to the metal body 10.

Regardless of the specific structure of the zinc-containing matrix, no matter how a relative positional relation between the zinc-containing matrix and the active drug layer changes according to the foregoing different embodiments, when the content of the active drug in the zinc-containing matrix per unit area is p (ug• mm⁻²), and the content of zinc corroded from the zinc-containing matrix per unit area in a guaranteed grade acetic acid aqueous solution at 8.3 vol% within unit time is p (µg•mm⁻²•min⁻¹), the value ofpand the value of p satisfy the following relation: 0.1≤15p+ρ≤3.5 where p is greater than or equal to 0.005 and less than or equal to 0.14.

By reasonably setting the content of the zinc and the content of the active drug on the zinc-containing matrix of the implantable drug-loaded device, the value of p and the value of p satisfy the above relation. It has been experimentally proven that the implantable drug-loaded device may control the release concentrations of zinc corrosion products and the active drug in a human body when it is implanted in the human body. The zinc and the drug work synergistically to inhibit smooth muscle cells, so that a lumen has a relatively good stenosis rate, and no cell apoptosis and tissue necrosis phenomena occur. In the corrosion process, the temperature of the guaranteed grade acetic acid aqueous solution at 8.3 vol.% is 25 °C, and the corrosion time is 20 minutes.

It can be noted that p and p in the above relation 0.1 ≦ 15p +ρ≦ 3.5 respectively refer to specific values, and units are not included in the calculation.

It can be understood that the fact that when the zinc-containing matrix is a matrix formed by the zinc or the zinc alloy, the content ρ (ug•mm⁻²) of the active drug in the zinc-containing matrix per unit area means that the content of the active drug in the matrix formed by the zinc or the zinc alloy of the unit area is ρ (ug•mm⁻²). The fact that when the zinc-containing matrix includes the body, and the zinc-containing portions attached to the body, the content of the active drug is ρ (µg•mm⁻²) means that the content of the active drug in the body of the unit area is p (µg•mm⁻²).

The surface area of the zinc-containing matrix refers to a total surface area of the zinc-containing matrix, i.e., a sum of the areas of the outer surface, the inner surface, and the side surface. The surface area of the zinc-containing matrix is a total surface area of the matrix formed of the pure zinc or the zinc alloy, or the surface area of the zinc-containing matrix refers to a total surface area of the body and the zinc-containing portions.

It can be noted that if the matrix formed by the pure zinc or the zinc alloy or the zinc-containing matrix is that a radiopaque structure is arranged on the body, and is filled with a radiopaque marker, an exposed area of the radiopaque marker is also considered as a part of the total surface area.

It can be noted that the reason why the guaranteed grade acetic acid aqueous solution at 8.3 vol.% at 25 °C is selected to corrode the zinc-containing matrix of the zinc-containing drug-loaded device for 20 minutes is as follows. At the present, by commercially available drug-loaded devices, the peaks of both the drug release rate and the tissue drug concentration appear within 1 month after the devices are implanted. It is necessary to ensure that the content of the zinc corroded from the zinc-containing matrix per unit area of the zinc-containing drug-loaded device within unit time is p_{in vivo} (µg•mm⁻²•min⁻¹) which avoids cell apoptosis and tissue necrosis phenomena occurring in a tissue in an implanted section. A result of an animal experiment on the zinc-containing drug-loaded device shows that the content p_{in vivo} (µg•mm⁻²•min⁻¹) of the zinc corroded from the zinc-containing matrix per unit area of the device in an animal body within the unit time and the content p of the zinc corroded from the zinc-containing matrix in the Guaranteed grade acetic acid aqueous solution at the concentration of 8.3 vol.% at 25 °C within the unit time are very nearly the same, so that p may represent pin vivo within one month after the zinc-containing drug-loaded device is implanted into the human body. Therefore, by testing p of the zinc-containing matrix of the zinc-containing implantable drug-loaded device in the guaranteed grade acetic acid aqueous solution at the concentration of 8.3 vol.% at 25 °C and combining the content p of the active drug on the zinc-containing matrix per unit area of the zinc-containing implantable drug-loaded device, whether the cell apoptosis and tissue necrosis phenomena occur in the tissue under the synergistic effect of the zinc and the drug after this implantable drug-loaded device is implanted into the human body may be determined.

By reasonably controlling the content p (µg•mm-2•min-1) of the zinc corroded from the zinc-containing matrix per unit area in the guaranteed grade acetic acid aqueous solution at 8.3 vol.% and the content ρ (ug•mm-2) of the active drug in the zinc-containing matrix per unit area, after zinc corrosion products and the active drug in the tissue reach certain concentrations, smooth muscle cell proliferation may be inhibited, and inflammatory reactions caused by extremely high concentrations of the zinc corrosion products and the active drug may not occur. Therefore, after the above-mentioned implantable drug-loaded device is implanted into the human body, the zinc and the drug work synergistically to play a role of inhibiting the smooth muscle cell proliferation without causing the cell apoptosis and the tissue necrosis.

It can be noted that in the above-mentioned implantable drug-loaded device, when the zinc-containing matrix is the matrix formed by the zinc alloy, or when the zinc-containing matrix includes the body, and the zinc-containing portions formed by the zinc alloy, regardless of the type of non-zinc elements in the zinc alloy, regardless of the content of the zinc and the content of the non-zinc elements in the zinc alloy, and regardless of a ratio of the active drug to the carrier in the active drug layer, the effect that the zinc and the drug may work synergistically to effectively inhibit the smooth muscle cell proliferation, and would not kill cells to cause the tissue necrosis may be achieved, as long as the non-zinc elements in the zinc alloy are harmless to the human body, or the content of the non-zinc elements in the zinc alloy is not enough to generate toxic action on the human body, and as long as the value of p and the value of p satisfy 0.1≤15p+ρ≤3.5 where p is greater than or equal to 0.005 and less than or equal to 0.14.

The implantable drug-loaded device is a vascular scaffold, a biliary scaffold, an esophageal scaffold, a urethral scaffold, an airway scaffold, an andrology implant, a gynecological implant, or a vena cava filter.

The implantable drug-loaded device is prepared by a method familiar to those of ordinary skill in the art.

For example, a laser cutting method is used to prepare the matrix formed by the pure zinc or the zinc alloy, and then a method such as spraying and soaking is used to form the active drug layer.

Or, the laser cutting method is used to prepare a body formed by a tubular product such as a degradable metal or a non-degradable metal, and then a method such as an electroplating method, a vapor deposition method and a vapor deposition method is used to form the zinc-containing portions on the body, and then prepare the active drug layer.

The implantable drug-loaded device is further described below through embodiments.

The following embodiments use the following detection methods.

### 1. Confirmation of zinc in the implantable drug-loaded device

Zinc is detected by X-ray photoelectron spectroscopy (XPS) or a scanning electron microscope (SEM). According to an actual position of the zinc in the zinc-containing matrix of the implantable drug-loaded device in the device, one section of a zinc-containing position of the implantable device is exposed by three methods, i.e., grinding after resin embedding, direct grinding and ion sputtering. Then, the section is observed by the XPS or the SEM. A square region is randomly selected in this section, and ingredients of the square region are detected through an accessory, i.e., an energy dispersion spectroscopy, of the XPS or the SEM, so as to determine this region as a constituent element containing the zinc and the zinc alloy.

The XPS is the ESCALAB 250Xi type XPS of Thermo Fisher Scientific, and the SEM is JSM6510 type SEM of Japan Electron Optics Laboratory.

### 2. Detection of the surface area of the zinc-containing matrix

If the zinc-containing matrix is of a regular shape, such as a cylindrical shape, dimensions may be detected directly by a macro test (a ruler and a micrometer caliper) or a micro test (a stereo microscope and the SEM), so as to calculate the surface area. If the zinc-containing matrix is of an irregular shape, micro-CT may be applied to scanning, so as to automatically calculate the surface area of the device.

### 3. Detection of a corrosion rate of the zinc of the implantable drug-loaded device

In all the embodiments according to the invention, the corrosion rate of the zinc-containing portions of the implantable drug-loaded device is tested in the guaranteed grade acetic acid aqueous solution at 8.3 vol.% at 25 °C according to the following method.

First, the active drug layer of the implantable drug-loaded device is eluted to expose the zinc-containing portions or expose the matrix formed of the pure zinc or the zinc alloy. After being weighed, the device is placed into the guaranteed grade acetic acid aqueous solution at 8.3 vol.% at 25 °C for 20 minutes. After the device is taken out, a fixed volume of acetic acid solution is used to fix the volume, and then digestion is performed. Atomic absorption spectroscopy (AAS) (e.g. with Agilent Spectr AA 240FS type atomic absorption spectroscopy) is used to test the zinc ion concentration in corrosive liquid, and the content of the zinc in the solution at this time is calculated, which is the zinc weight loss of the zinc-containing portions or the matrix formed by the pure zinc or the zinc alloy. Then, the content is divided by the surface area of the device and the corrosion time to obtain the content p of the zinc corroded from the zinc-containing matrix per unit area in the guaranteed grade acetic acid aqueous solution at 8.3 vol.% within unit time.

### 4. Detection of the active drug in the implantable drug-loaded device

The implantable drug-loaded device to be tested is put into a brown glass bottle, and an organic solvent that may dissolve the active drug is quantitatively added according to the active drug carried by it. The sample bottle with a fixed volume is placed in an ultrasonic instrument for 20 minutes. After the sample bottle subjected to the ultrasonic treatment is recovered to room temperature, an extracting solution is sucked with an injector, and is filtered with a 0.22µm organic filter. The extracting solution is filtered into a 2mL sample injection bottle as a test sample solution which is tested by using a liquid chromatograph.

The liquid chromatograph is Agilent 1260.

### 5. Stenosis rate detection and pathological observation in the tissue surrounding the implantable drug-loaded device

The stenosis rate in the tissue surrounding the implantable drug-loaded device is confirmed by an animal implantation experiment. The vascular scaffold is taken as an example. The following steps are included: the vascular scaffold is implanted into a blood vessel of a test animal. Then, at predetermined observation time points, such as one month and three months, the blood vessel at a scaffold section is taken out for fixation, embedding, sectioning, and staining to make a pathological section. The histopathology is observed by using the DM2500 type microscope of German LEICA company, and a lumen area of a cross section where the blood vessel at the scaffold section is located and an original lumen area are measured. Then, there is: lumen stenosis rate = (original lumen area-existing lumen area)/original lumen area X 100%.

### Embodiment 1

Zinc was uniformly plated on the surface of an iron-alloy vascular scaffold body having a surface area of 34.6 mm² by using an electroplating method, so as to form a zinc plated layer on the surface of the iron-alloy vascular scaffold body. Then, a rapamycin and polylactic acid mixture was uniformly sprayed to the surface of the zinc plated layer. After the surface was dried, a rapamycin-polylactic acid coating was formed on the surface of the zinc plated layer, thus obtaining the vascular scaffold of the present embodiment. The zinc plated layer in the present embodiment was the zinc-containing portions. The zinc-containing matrix was composed of the iron-alloy vascular scaffold body and the zinc plated layer.

Through the above-mentioned detection method, it was measured that the content p of the rapamycin in the zinc-containing matrix per unit area was 1.4 ug•mm⁻², and the content p of the zinc corroded from the zinc-containing matrix per unit area in the guaranteed grade acetic acid aqueous solution at 8.3 vol.% at 25 °C within the unit time was 0.065 ug•mm⁻²•min⁻¹, 15p+p=2.375.

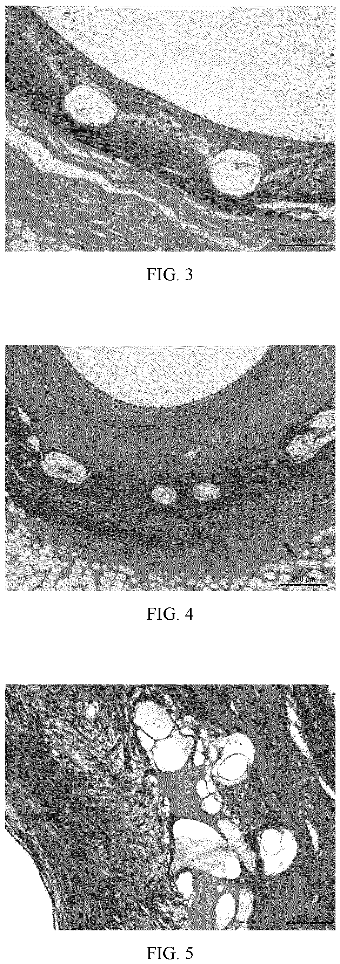

The vascular scaffold in the present embodiment was implanted into a coronary vessel of a minipig, and an over-expanding ratio range was kept at 1.1:1 to 1.2:1 in the implantation process. Then, follow-up were carried out within three months after the implantation. During the follow-up, the vascular scaffold and vascular tissues around the vascular scaffold were taken out to perform pathologic analysis on the tissues around the vascular scaffold, and a pathological image was as shown in FIG. 3. A pathologic analysis result showed that after the vascular scaffold provided by Embodiment 1 was implanted into the animal body for three months, no obvious smooth muscle cell proliferation phenomenon occurred in the tissues around the scaffold, endothelial cells of vascular tissues grew normally, and no histocyte necrosis occurred around a scaffold strut. The stenosis rate of the blood vessel was measured as 27% through the above-mentioned detection method.

### Embodiment 2

A pure iron vascular scaffold body having a surface area of 34.6 mm² was soaked into a zinc plating solution in half of a lengthwise direction. Zinc was uniformly plated by using an electroplating method, so as to form a zinc plated layer on half of the surface of the pure iron vascular scaffold body. Then, the zinc-plated pure iron vascular scaffold body was placed in a vacuum furnace for thermal treatment at furnace chamber pressure of 20 Pa and thermal treatment temperature of 300 °C for 3 h. After the thermal treatment was completed, the sample was taken out, and was naturally cooled to room temperature. A rapamycin and polylactic acid mixture was uniformly sprayed to the surface of the scaffold body. After the surface was dried, a rapamycin-polylactic acid coating was formed on the surface of the scaffold body, thus obtaining the vascular scaffold of the present embodiment. In the present embodiment, after the thermal treatment, a zinc-iron alloy layer covering the surface of the pure iron vascular scaffold body was formed. The zinc-iron alloy layer was the zinc-containing portions. The zinc-containing matrix was composed of the pure iron vascular scaffold body and the zinc-iron alloy layer.

Through the above-mentioned detection method, it was measured that the contentpof the rapamycin in the zinc-containing matrix per unit area was 2.3 ug•mm⁻², and the content p of the zinc corroded from the zinc-containing matrix per unit area in the guaranteed grade acetic acid aqueous solution at 8.3 vol.% at 25 °C within the unit time was 0.005 ug•mm⁻²•min⁻¹, 15p+p=2.375.

The vascular scaffold in the present embodiment was implanted into a coronary vessel of a minipig, and an over-expanding ratio range was kept at 1:1 to 1.2:1 in the implantation process. Then, follow-up were carried out within three months after the implantation. During the follow-up, the vascular scaffold and vascular tissues around the vascular scaffold were taken out to perform pathologic analysis on the tissues around the vascular scaffold. A pathologic analysis result showed that after the vascular scaffold provided by Embodiment 2 was implanted into the animal body for three months, no obvious smooth muscle cell proliferation phenomenon occurred in the tissues around the scaffold, endothelial cells of vascular tissues grew normally, and no histocyte necrosis occurred around a scaffold strut. The stenosis rate of the blood vessel was measured as 30% through the above-mentioned detection method.

### Embodiment 3

Zinc was uniformly plated on the surface of an iron-alloy vascular scaffold body having a surface area of 34.6 mm² by using an electroplating method, so as to form a zinc plated layer on the surface of the iron-alloy vascular scaffold body. Then, a rapamycin and polylactic acid mixture was uniformly sprayed to the surface of the zinc plated layer. After the surface was dried, a rapamycin-polylactic acid coating was formed on the surface of the zinc plated layer, thus obtaining the vascular scaffold of the present embodiment. The zinc plated layer in the present embodiment was the zinc-containing portions. The zinc-containing matrix was composed of the iron-alloy scaffold body, and the zinc plated layer.

Through the above-mentioned detection method, it was measured that the content p of the rapamycin in the zinc-containing matrix per unit area was 0.5 ug•mm⁻², and the content p of the zinc corroded from the zinc-containing matrix per unit area in the guaranteed grade acetic acid aqueous solution at 8.3 vol.% at 25 °C within the unit time was 0.14 ug•mm⁻²•min⁻¹, 15p+p=2.6.

The vascular scaffold in the present embodiment was implanted into a coronary vessel of a minipig, and an over-expanding ratio range was kept at 1.1:1 to 1.2:1 in the implantation process. Then, follow-ups were carried out within three months after the implantation. During the follow-up, the vascular scaffold and vascular tissues around the vascular scaffold were taken out to perform pathologic analysis on the tissues around the vascular scaffold. A result showed that after the vascular scaffold provided by Embodiment 3 was implanted into the animal body for three months, no obvious smooth muscle cell proliferation phenomenon occurred in the tissues around the vascular scaffold, endothelial cells of vascular tissues grew normally, and no histocyte necrosis occurred around a scaffold strut. The stenosis rate of the blood vessel was measured as 32% through the above-mentioned detection method.

### Embodiment 4

A magnesium-alloy vascular scaffold body having a surface area of 34.6 mm² was soaked into a zinc plating solution in half of a lengthwise direction. Zinc was uniformly plated by using an electroplating method, so as to form a zinc plated layer on half of the surface of the magnesium-alloy vascular scaffold body. Then, after the zinc plated layer was covered, a rapamycin and polylactic acid mixture was uniformly sprayed to the surface of the magnesium-alloy vascular scaffold body. After the surface was dried, a rapamycin-polylactic acid coating was formed on the surface of the magnesium-alloy vascular scaffold body, and the zinc plated layer and the rapamycin-polylactic acid coating have no overlapping regions, thus obtaining the vascular scaffold of the present embodiment. The zinc plated layer in the present embodiment was the zinc-containing portions. The zinc-containing matrix was composed of the magnesium-alloy vascular scaffold body and the zinc plated layer.

Through the above-mentioned detection method, it was measured that the content p of the rapamycin in the zinc-containing matrix per unit area was 1.15 ug•mm⁻², and the content p of the zinc corroded from the zinc-containing matrix per unit area in the guaranteed grade acetic acid aqueous solution at 8.3 vol.% at 25 °C within the unit time was 0.1 ug•mm⁻²•min⁻¹, 15p+p=2.65.

The vascular scaffold in the present embodiment was implanted into a coronary vessel of a minipig, and an over-expanding ratio range was kept at 1:1 to 1.2:1 in the implantation process. Then, follow-ups were carried out within three months after the implantation. During the follow-up, the scaffold and vascular tissues around the scaffold were taken out to perform pathologic analysis on the tissues around the scaffold. A result showed that after the vascular scaffold provided by Embodiment 4 was implanted into the animal body for three months, no obvious smooth muscle cell proliferation phenomenon occurred in the tissues around the vascular scaffold, endothelial cells of vascular tissues grew normally, and no histocyte necrosis occurred around a scaffold strut. The stenosis rate of the blood vessel was measured as 35% through the above-mentioned detection method.

### Embodiment 5

A zinc-iron alloy layer was electrically plated on the surface of a 316L stainless steel vascular scaffold having a surface area of 34.6 mm². An atomic ratio of zinc to iron in the zinc-iron alloy layer was 7:3. Then, a paclitaxel and polylactic acid mixture was uniformly sprayed to the zinc-iron alloy layer. After the zinc-iron alloy layer was dried, the vascular scaffold of the present embodiment was obtained. The zinc-iron alloy layer in the present embodiment was the zinc-containing portions. The zinc-containing matrix was composed of the 316L stainless steel vascular scaffold body, and the zinc-iron alloy layer.

Through the above-mentioned detection method, it was measured that the content p of the paclitaxel in the zinc-containing matrix per unit area was 0.025 ug•mm⁻², and the content p of the zinc corroded from the zinc-containing matrix per unit area in the guaranteed grade acetic acid aqueous solution at 8.3 vol.% within the unit time was 0.005 ug•mm⁻²•min⁻¹, 15p+p=0.1.

The vascular scaffold in the present embodiment was implanted into a coronary vessel of a minipig, and an over-expanding ratio range was kept at 1.1:1 to 1.2:1 in the implantation process. Then, follow-ups were carried out within three months after the implantation. During the follow-up, the vascular scaffold and vascular tissues around the vascular scaffold were taken out to perform pathologic analysis on the tissues around the vascular scaffold. A pathologic analysis result showed that after the vascular scaffold provided by Embodiment 5 was implanted into the animal body for three months, no obvious smooth muscle cell proliferation phenomenon occurred in the tissues around the scaffold, endothelial cells of vascular tissues grew normally, and no histocyte necrosis occurred around a scaffold strut. The stenosis rate of the blood vessel was measured as 36% through the above-mentioned detection method.

### Embodiment 6

A strut wall of a polylactic acid vascular scaffold body having a surface area of 34.6 mm² was provided with grooves, and the grooves in the surface of the polylactic acid vascular scaffold body were filled with pure zinc powder. Then, a rapamycin and polylactic acid mixture was uniformly sprayed to the surface of the polylactic acid vascular scaffold body. After the surface was dried, a rapamycin-polylactic acid coating was formed, thus obtaining the vascular scaffold of the present embodiment. In the present embodiment, the grooves of the polylactic acid vascular scaffold body were filled with the pure zinc powder to form the zinc-containing portions arranged on the polylactic acid vascular scaffold body.

Through the above-mentioned detection method, it was measured that the content p of the rapamycin in the zinc-containing matrix per unit area was 0.69 ug•mm⁻², and the content p of the zinc corroded from the zinc-containing matrix per unit area in the guaranteed grade acetic acid aqueous solution at 8.3 vol.% within the unit time was 0.12 ug•mm⁻²•min⁻¹, 15p+p=2.49.

The vascular scaffold in the present embodiment was implanted into a coronary vessel of a minipig, and an over-expanding ratio range was kept at 1:1 to 1.2:1 in the implantation process. Then, follow-up were carried out within three months after the implantation. During the follow-up, the vascular scaffold and vascular tissues around the vascular scaffold were taken out to perform pathologic analysis on the tissues around the vascular scaffold. A pathologic analysis result showed that after the vascular scaffold provided by Embodiment 6 was implanted into the animal body for three months, no obvious smooth muscle cell proliferation phenomenon occurred in the tissues around the scaffold, endothelial cells of vascular tissues grew normally, and no histocyte necrosis occurred around a scaffold strut. The stenosis rate of the blood vessel was measured as 33% through the above-mentioned detection method.

### Embodiment 7

A rapamycin and polylactic acid mixture was uniformly sprayed to the outer surface of a pure zinc vascular scaffold matrix having a surface area of 34.6 mm². After the outer surface was dried, a rapamycin-polylactic acid coating was formed on the outer surface of the pure zinc vascular scaffold matrix, thus obtaining the vascular scaffold of the present embodiment. In the present embodiment, the pure zinc vascular scaffold matrix was the zinc-containing matrix.

Through the above-mentioned detection method, it was measured that the content p of the rapamycin in the zinc-containing matrix per unit area was 1.4 ug•mm⁻², and the content p of the zinc corroded from the zinc-containing matrix per unit area in the Guaranteed grade acetic acid aqueous solution at 8.3 vol.% at 25 °C within the unit time was 0.035 ug•mm⁻²•min⁻¹, 15p+p=1.925.

The vascular scaffold in the present embodiment was implanted into a coronary vessel of a minipig, and an over-expanding ratio range was kept at 1.1:1 to 1.2:1 in the implantation process. Then, follow-ups were carried out within three months after the implantation. During the follow-up, the scaffold and vascular tissues around the scaffold were taken out to perform pathologic analysis on the tissues around the scaffold A result showed that after the vascular scaffold provided by Embodiment 7 was implanted into the animal body for three months, no obvious smooth muscle cell proliferation phenomenon occurred in the tissues around the vascular scaffold, endothelial cells of vascular tissues grew normally, and no histocyte necrosis occurred around a scaffold strut. The stenosis rate of the blood vessel was measured as 36% through the above-mentioned detection method.

### Embodiment 8

A rapamycin and polylactic acid mixture was uniformly sprayed to the surface of a zinc-alloy vascular scaffold matrix having a surface area of 34.6 mm². After the surface was dried, a rapamycin-polylactic acid coating was formed on the surface of the zinc-alloy vascular scaffold matrix, thus obtaining the vascular scaffold of the present embodiment. In the present embodiment, the zinc-alloy vascular scaffold matrix was the zinc-containing matrix.

Through the above-mentioned detection method, it was measured that the content p of the rapamycin in the zinc-containing matrix per unit area was 2.3 ug•mm⁻², and the content p of the zinc corroded from the zinc-containing matrix per unit area in the guaranteed grade acetic acid aqueous solution at 8.3 vol.% at 25 °C within the unit time was 0.08 ug•mm⁻²•min⁻¹, 15p+p=3.5.

The vascular scaffold in the present embodiment was implanted into a coronary vessel of a minipig, and an over-expanding ratio range was kept at 1:1 to 1.2:1 in the implantation process. Then, follow-up were carried out within three months after the implantation. During the follow-up, the vascular scaffold and vascular tissues around the vascular scaffold were taken out to perform pathologic analysis on the tissues around the vascular scaffold. A pathologic analysis result showed that after the vascular scaffold provided by Embodiment 8 was implanted into the animal body for three months, no obvious smooth muscle cell proliferation phenomenon occurred in the tissues around the scaffold, endothelial cells of vascular tissues grew normally, and no histocyte necrosis occurred around a scaffold strut. The stenosis rate of the blood vessel was measured as 32% through the above-mentioned detection method.

### Comparative example 1

A zinc-iron alloy layer was electrically plated on the surface of a 316L stainless steel vascular scaffold having a surface area of 34.6 mm². An atomic ratio of zinc to iron in the zinc-iron alloy layer was 7:3. Then, a paclitaxel and polylactic acid mixture was uniformly sprayed to the zinc-iron alloy layer. After the zinc-iron alloy layer was dried, the vascular scaffold of the present embodiment was obtained. The zinc-iron alloy layer in the present embodiment was the zinc-containing portions. The zinc-containing matrix was composed of the stainless steel vascular scaffold body and the zinc-iron alloy layer.

Through the above-mentioned detection method, it was measured that the content p of the rapamycin in the zinc-containing matrix per unit area was 0.01 ug•mm-2, and the content p of the zinc corroded from the zinc-containing matrix per unit area in the Guaranteed grade acetic acid aqueous solution at 8.3 vol.% at 25 °C within the unit time was 0.005 ug•mm⁻²•min⁻¹, 15p+p=0.085.

The vascular scaffold in the present comparative example was implanted into a coronary vessel of a minipig, and an over-expanding ratio range was kept at 1.1:1 to 1.2:1 in the implantation process. Then, follow-ups were carried out within three months after the implantation. During the follow-up, the vascular scaffold and vascular tissues around the vascular scaffold were taken out to perform pathologic analysis on the tissues around the vascular scaffold, and a pathological image was as shown in FIG. 4. A pathologic analysis result showed that after the vascular scaffold provided by the comparative example 1 was implanted into the animal body for three months, no obvious smooth muscle cell proliferation phenomenon occurred in the tissues around the scaffold, endothelial cells of vascular tissues grew normally, and no histocyte necrosis occurred around a scaffold strut. Through the above-mentioned detection method, the stenosis rate of the blood vessel was measured as 45%, which is obviously more than that of Embodiment 5.

### Comparative example 2

A pure iron vascular scaffold body having a surface area of 34.6 mm² was soaked into a zinc plating solution in half of a lengthwise direction. Zinc was uniformly plated by using an electroplating method, so as to form a zinc plated layer on the surface of half of a matrix of the pure iron vascular scaffold. Then, the zinc-plated pure iron vascular scaffold body was placed in a vacuum furnace for thermal treatment at furnace chamber pressure of 20 Pa and thermal treatment temperature of 300 °C for 3 h. After the thermal treatment was completed, the sample was taken out, and was naturally cooled to room temperature. A rapamycin and polylactic acid mixture was uniformly sprayed to the surface of the scaffold body. After the surface was dried, a rapamycin-polylactic acid coating was formed on the surface of the scaffold body, thus obtaining the vascular scaffold of the present embodiment. In the present embodiment, after the thermal treatment, a zinc-iron alloy layer covering the surface of the pure iron vascular scaffold body was formed. The zinc-iron alloy layer was the zinc-containing portions. The zinc-containing matrix was composed of the pure iron vascular scaffold body and the zinc-iron alloy layer.

Through the above-mentioned detection method, it was measured that the content p of the rapamycin in the zinc-containing matrix per unit area was 3.6 ug•mm⁻², and the content p of the zinc corroded from the zinc-containing matrix per unit area in the guaranteed guade acetic acid aqueous solution at 8.3 vol.% at 25 °C within the unit time was 0.005 ug•mm⁻²•min⁻¹, 15p+p=3.675.

The vascular scaffold in the present comparative example was implanted into a coronary vessel of a minipig, and an over-expanding ratio range was kept at 1.1:1 to 1.2:1 in the implantation process. Then, follow-ups were carried out within one month after the implantation. During the follow-up, the vascular scaffold and vascular tissues around the vascular scaffold were taken out to perform pathologic analysis on the tissues around the vascular scaffold, and a pathological image was as shown in FIG. 5. A pathologic analysis result showed that after the vascular scaffold provided by the comparative example 2 was implanted for one month, obvious intimal hyperplasia occurred around a scaffold strut, obvious luminal stenosis occurred in the blood vessel where the scaffold was placed, and obvious inflammatory cells appeared around the scaffold strut.

The result shows that compared with the vascular scaffold provided by Embodiment 2, the vascular scaffold of the comparative example 2 loads excessive drugs, resulting in necrocytosis in the tissues around the scaffold.

### Comparative example 3

Zinc was uniformly plated on the surface of an iron-alloy vascular scaffold body having a surface area of 34.6 mm² by using an electroplating method, so as to form a zinc plated layer on the surface of an iron-alloy vascular scaffold matrix. Then, a rapamycin and polylactic acid mixture was uniformly sprayed to the surface of the zinc plated layer. After the surface was dried, a rapamycin-polylactic acid coating was formed on the surface of the zinc plated layer, thus obtaining the vascular scaffold of the present embodiment. In the present embodiment, the zinc plated layer was the zinc-containing portions. The zinc-containing matrix was composed of the iron-alloy vascular scaffold body and the zinc plated layer.

Through the above-mentioned detection method, it was measured that the content p of the rapamycin in the zinc-containing matrix per unit area was 0.5 ug•mm⁻², and the content p of the zinc corroded from the zinc-containing matrix per unit area in the guaranteed grade acetic acid aqueous solution at 8.3 vol.% at 25 °C within the unit time was 0.22 ug•mm⁻²•min⁻¹, 15p+p=3.8.

The vascular scaffold in the present comparative example was implanted into a coronary vessel of a minipig, and an over-expanding ratio range was kept at 1.1:1 to 1.2:1 in the implantation process. Then, follow-up were carried out within one month after the implantation. During the follow-up, the vascular scaffold and vascular tissues around the vascular scaffold were taken out to perform pathologic analysis on the tissues around the vascular scaffold. A result showed that after the vascular scaffold provided by the comparative example 3 was implanted for one month, obvious intimal hyperplasia occurred around a scaffold strut, obvious luminal stenosis occurred in the blood vessel where the scaffold was placed, and obvious inflammatory cells appeared around the scaffold strut.

The result shows that compared with the vascular scaffold provided by Embodiment 3, the vascular scaffold of the comparative example 3 causes necrocytosis in the tissues around the scaffold due to too fast corrosion of the zinc-containing portions.

## Claims

1. An implantable drug-loaded device, comprising:
a zinc-containing matrix and an active drug layer attached to the zinc-containing matrix, wherein in the zinc-containing matrix per unit area, the content of an active drug is p,having the unit of µg•mm⁻²; the content of zinc corroded from the zinc-containing matrix per unit area in a guaranteed grade acetic acid aqueous solution at 8.3 vol.% within unit time is p, having the unit of µg•mm⁻²•min⁻¹, wherein the content of zinc corroded from the zinc-containing matrix per unit area is tested in the guaranteed grade acetic acid aqueous solution at 8.3 vol.% at 25 °C by a method consisting of the following steps:
- firstly, eluting the active drug layer of the implantable drug-loaded device to expose the zinc-containing portions or to expose the matrix formed of the pure zinc or the zinc alloy;
- after being weighed, placing the device into the guaranteed grade acetic acid aqueous solution at 8.3 vol.% at 25 °C for 20 minutes;
- after taking out the device, using a fixed volume of acetic acid solution to fix the volume, and then performing a digestion;
- testing the zinc ion concentration in corrosive liquid with atomic absorption spectroscopy;
- calculating the content of the zinc in the solution at this time, which is the zinc weight loss of the zinc-containing portions or the matrix formed by the pure zinc or the zinc alloy;
- finally, dividing the content by the surface area of the device and the corrosion time to obtain the content p of the zinc corroded from the zinc-containing matrix per unit area in the guaranteed grade acetic acid aqueous solution at 8.3 vol.% within unit time;
wherein the *ρ* and the p satisfy the following relation:
0.1≤15p+ρ≤3.5, where p is greater than or equal to 0.005 and less than or equal to 0.14.

2. The implantable drug-loaded device according to claim 1, wherein the zinc-containing matrix is a matrix formed by zinc or a zinc alloy; or the zinc-containing matrix comprises a body and zinc-containing portions attached to the body; and
a material of the zinc-containing portions is pure zinc or a zinc alloy.

3. The implantable drug-loaded device according to claim 2, wherein the zinc alloy is an alloy formed by zinc and at least one of iron, magnesium, palladium, rhenium, gold, platinum, tantalum, and strontium; or the zinc alloy is an alloy formed by doping zinc with at least one of carbon, nitrogen, oxygen, sulfur, boron, and silicon.

4. The implantable drug-loaded device according to claim 2, wherein when the zinc-containing matrix comprises the body, and the zinc-containing portions attached to the body, and the material of the zinc-containing portions is the pure zinc or the zinc alloy, the zinc-containing portions at least partially cover the surface of the body, or the zinc-containing portions fill the inside of the body.

5. The implantable drug-loaded device according to claim 2, wherein when the zinc-containing matrix comprises the body and the zinc-containing portions attached to the body, and the material of the zinc-containing portions is the zinc alloy, the body is a metal body, and the zinc-containing portions are formed by reaction between the metal body and pure zinc.

6. The implantable drug-loaded device according to claim 2, wherein when the zinc-containing matrix comprises the body and the zinc-containing portions attached to the body, and the material of the zinc-containing portions is the pure zinc or the zinc alloy, the active drug layer does not cover the zinc-containing portions at all, or the active drug layer at least partially covers the zinc-containing portions.

7. The implantable drug-loaded device according to claim 1, wherein the active drug layer comprises an active drug and a carrier, and the active drug is dispersed in the carrier.

8. The implantable drug-loaded device according to claim 7, wherein the active drug is at least one of heparin, colchicine, rapamycin and a derivative thereof, paclitaxel and a derivative thereof, and hirudin and a derivative thereof.

9. The implantable drug-loaded device according to claim 7, wherein the carrier is at least one of a degradable polymer, a non-degradable polymer, and a copolymer formed by at least two of monomers that form the degradable polymer and monomers that form the non-degradable polymer; and the degradable polymer is at least one of polylactic acid, polyglycolic acid, polycaprolactone, polysuccinate, and poly(β-hydroxybutyrate), and the non-degradable polymer is at least one of polystyrene, polytetrafluoroethylene, polymethyl methacrylate, polycarbonate, and polyethylene terephthalate.

10. The implantable drug-loaded device according to claim 2, wherein the body is made of a metal, and the metal is a degradable metal or a non-degradable metal.

11. The implantable drug-loaded device according to claim 10, wherein the degradable metal is pure iron, an iron alloy, pure magnesium, or a magnesium alloy.

12. The implantable drug-loaded device according to claim 10, wherein the non-degradable metal is 316L stainless steel, a cobalt-chromium alloy, a platinum-chromium alloy, pure titanium or a titanium alloy.

13. The implantable drug-loaded device according to claim 2, wherein the body is at least partially made of a polymer; the polymer is at least one of a degradable polymer, a non-degradable polymer, and a copolymer formed by at least two of monomers that form the degradable polymer and monomers that form the non-degradable polymer; the degradable polymer is at least one of polylactic acid, polyglycolic acid, polycaprolactone, polysuccinate, and poly(β-hydroxybutyrate), and the non-degradable polymer is at least one of polystyrene, polytetrafluoroethylene, polymethyl methacrylate, polycarbonate, and polyethylene terephthalate.

14. The implantable drug-loaded device according to claim 1, wherein the implantable drug-loaded device is a vascular scaffold, a biliary scaffold, an esophageal scaffold, a urethral scaffold, an airway scaffold, an andrology implant, a gynecological implant, or a vena cava filter.

## Patentansprüche

1. Implantierbare arzneimittelbeladene Vorrichtung, die Folgendes umfasst:
eine zinkhaltige Matrix und eine an die zinkhaltige Matrix angelagerte Arzneimittelwirkstoffschicht, wobei in der zinkhaltigen Matrix pro Flächeneinheit der Gehalt an einem Arzneimittelwirkstoff *ρ* mit der Einheit µg•mm⁻² ist, der Gehalt an Zink, das aus der zinkhaltigen Matrix pro Flächeneinheit in einer wässrigen Lösung von Essigsäure garantierten Gütegrades bei 8,3 Vol.-% innerhalb einer Zeiteinheit korrodiert, p mit der Einheit µg•mm⁻²•min⁻¹ ist, wobei der Gehalt an aus der zinkhaltigen Matrix pro Flächeneinheit korrodiertem Zink in der wässrigen Lösung von Essigsäure garantierten Gütegrades bei 8,3 Vol.-% bei 25°C durch ein Verfahren geprüft wird, das aus den folgenden Schritten besteht:
- zunächst wird die Arzneimittelwirkstoffschicht der implantierbaren arzneimittelbeladenen Vorrichtung eluiert, um die zinkhaltigen Bereiche oder die aus dem reinen Zink oder der Zinklegierung gebildete Matrix freizulegen;
- nach dem Wiegen wird die Vorrichtung 20 Minuten lang in die wässrige Lösung von Essigsäure garantierten Gütegrades bei 8,3 Vol.-% bei 25°C gelegt;
- nach dem Herausnehmen der Vorrichtung wird ein festgelegtes Volumen an Essigsäurelösung zur Fixierung des Volumens verwendet, und dann wird ein Aufschluss durchgeführt;
- die Zinkionenkonzentration in korrosiver Flüssigkeit wird mittels Atomabsorptionsspektroskopie geprüft;
- der Gehalt des Zinks in der Lösung zu diesem Zeitpunkt wird berechnet, der dem Zinkgewichtsverlust der zinkhaltigen Bereiche oder der durch das reine Zink oder die Zinklegierung gebildeten Matrix entspricht;
- schließlich wird der Gehalt durch den Flächeninhalt der Vorrichtung und die Korrosionszeit dividiert, um den Gehalt p des aus der zinkhaltigen Matrix korrodierten Zinks pro Flächeneinheit in der wässrigen Lösung von Essigsäure garantierten Gütegrades bei 8,3 Vol.-% innerhalb der Zeiteinheit zu erhalten;
wobei p und p die folgende Beziehung erfüllen:
0,1≤15p+ρ≤3,5, worin p größer oder gleich 0,005 und kleiner oder gleich 0,14 ist.

2. Implantierbare arzneimittelbeladene Vorrichtung nach Anspruch 1, bei der die zinkhaltige Matrix eine aus Zink oder einer Zinklegierung gebildete Matrix ist oder die zinkhaltige Matrix einen Körper und an den Körper angelagerte zinkhaltige Bereiche umfasst, und
ein Material der zinkhaltigen Bereiche reines Zink oder eine Zinklegierung ist.

3. Implantierbare arzneimittelbeladene Vorrichtung nach Anspruch 2, bei der die Zinklegierung eine aus Zink und Eisen und/oder Magnesium und/oder Palladium und/oder Rhenium und/oder Gold und/oder Platin und/oder Tantal und/oder Strontium gebildete Legierung ist oder die Zinklegierung eine durch Dotierung von Zink mit Kohlenstoff und/oder Stickstoff und/oder Sauerstoff und/oder Schwefel und/oder Bor und/oder Silizium gebildete Legierung ist.

4. Implantierbare arzneimittelbeladene Vorrichtung nach Anspruch 2, bei der dann, wenn die zinkhaltige Matrix den Körper und die an den Körper angelagerten zinkhaltigen Bereiche umfasst und das Material der zinkhaltigen Bereiche das reine Zink oder die Zinklegierung ist, die zinkhaltigen Bereiche zumindest teilweise die Oberfläche des Körpers bedecken oder die zinkhaltigen Bereiche das Innere des Körpers ausfüllen.

5. Implantierbare arzneimittelbeladene Vorrichtung nach Anspruch 2, bei der dann, wenn die zinkhaltige Matrix den Körper und die an den Körper angelagerten zinkhaltigen Bereiche umfasst und das Material der zinkhaltigen Bereiche die Zinklegierung ist, der Körper ein Metallkörper ist und die zinkhaltigen Bereiche durch Reaktion zwischen dem Metallkörper und reinem Zink gebildet sind.

6. Implantierbare arzneimittelbeladene Vorrichtung nach Anspruch 2, bei der dann, wenn die zinkhaltige Matrix den Körper und die an den Körper angelagerten zinkhaltigen Bereiche umfasst und das Material der zinkhaltigen Bereiche das reine Zink oder die Zinklegierung ist, die Arzneimittelwirkstoffschicht die zinkhaltigen Bereiche überhaupt nicht bedeckt oder die Arzneimittelwirkstoffschicht die zinkhaltigen Bereiche zumindest teilweise bedeckt.

7. Implantierbare arzneimittelbeladene Vorrichtung nach Anspruch 1, bei der die Arzneimittelwirkstoffschicht einen Arzneimittelwirkstoff und einen Träger umfasst und der Arzneimittelwirkstoff in dem Träger dispergiert ist.

8. Implantierbare arzneimittelbeladene Vorrichtung nach Anspruch 7, bei der der Arzneimittelwirkstoff Heparin und/oder Colchicin und/oder Rapamycin und/oder ein Derivat davon und/oder Paclitaxel und/oder ein Derivat davon und/oder Hirudin und/oder ein Derivat davon ist.

9. Implantierbare arzneimittelbeladene Vorrichtung nach Anspruch 7, bei der der Träger ein abbaubares Polymer und/oder ein nicht abbaubares Polymer und/oder ein Copolymer ist, das aus mindestens zwei von Monomeren, die das abbaubare Polymer bilden, und Monomeren gebildet ist, die das nicht abbaubare Polymer bilden, und das abbaubare Polymer Polymilchsäure und/oder Polyglykolsäure und/oder Polycaprolacton und/oder Polysuccinat und/oder Poly(β-hydroxybutyrat) ist und das nicht abbaubare Polymer Polystyrol und/oder Polytetrafluorethylen und/oder Polymethylmethacrylat und/oder Polycarbonat und/oder Polyethylenterephthalat ist.

10. Implantierbare arzneimittelbeladene Vorrichtung nach Anspruch 2, bei der der Körper aus einem Metall besteht und das Metall ein abbaubares Metall oder ein nicht abbaubares Metall ist.

11. Implantierbare arzneimittelbeladene Vorrichtung nach Anspruch 10, bei der das abbaubare Metall reines Eisen, eine Eisenlegierung, reines Magnesium oder eine Magnesiumlegierung ist.

12. Implantierbare arzneimittelbeladene Vorrichtung nach Anspruch 10, bei der das nicht abbaubare Metall Edelstahl 316L, eine Kobalt-Chrom-Legierung, eine Platin-Chrom-Legierung, reines Titan oder eine Titanlegierung ist.

13. Implantierbare arzneimittelbeladene Vorrichtung nach Anspruch 2, bei der der Körper zumindest teilweise aus einem Polymer besteht, das Polymer ein abbaubares Polymer und/oder ein nicht abbaubares Polymer und/oder ein Copolymer ist, das aus mindestens zwei von Monomeren, die das abbaubare Polymer bilden, und Monomeren gebildet ist, die das nicht abbaubare Polymer bilden, das abbaubare Polymer Polymilchsäure und/oder Polyglykolsäure und/oder Polycaprolacton und/oder Polysuccinat und/oder Poly(β-hydroxybutyrat) ist und das nicht abbaubare Polymer Polystyrol und/oder Polytetrafluorethylen und/oder Polymethylmethacrylat und/oder Polycarbonat und/oder Polyethylenterephthalat ist.

14. Implantierbare arzneimittelbeladene Vorrichtung nach Anspruch 1, wobei die implantierbare arzneimittelbeladene Vorrichtung ein Gefäßgerüst, ein biliäres Gerüst, ein Speiseröhrengerüst, ein Harnröhrengerüst, ein Atemwegsgerüst, ein andrologisches Implantat, ein gynäkologisches Implantat oder ein Hohlvenenfilter ist.

## Revendications

1. Dispositif implantable chargé de médicaments, comprenant :
une matrice contenant du zinc et un couche de médicament actif attachée à la matrice contenant du zinc, dans lequel dans la matrice contenant du zinc, par unité de surface, la teneur en un médicament actif est *ρ* d'unité ug•mm⁻² ; la teneur en zinc se corrodant de la matrice contenant du zinc, par unité de surface, dans une solution aqueuse d'acide acétique de qualité garantie à 8,3% en volume dans l'unité de temps est p d'unité ug•mm⁻²•min⁻¹, dans lequel la teneur en zinc se corrodant de la matrice contenant du zinc, par unité de surface, est contrôlée dans la solution aqueuse d'acide acétique de qualité garantie à 8,3% en volume à 25°C au moyen d'un procédé qui comprend les étapes suivantes :
- tout d'abord l'élution de la couche de médicament actif du dispositif implantable chargé de médicaments pour exposer les zones contentant du zinc ou pour exposer la matrice formée par le zinc pur ou l'alliage de zinc ;
- l'agencement, après la pesée, du dispositif dans la solution aqueuse d'acide acétique de qualité garantie à 8,3% en volume à 25°C pendant 20 minutes ;
- après le retrait du dispositif, l'utilisation d'un volume fixe de la solution d'acide acétique pour fixer le volume, puis la réalisation d'une dissolution ;
- le contrôle de la concentration d'ions de zinc dans un liquide corrosif par spectroscopie d'absorption atomique ;
- le calcul de la teneur en zinc dans la solution à cet instant correspondant à la perte de poids en zinc des zones contenant du zinc ou la matrice formée par le zinc pur ou l'alliage de zinc ;
- finalement la division de la teneur par la superficie du dispositif et le temps de corrosion pour obtenir la teneur p en zinc se corrodant de la matrice contenant du zinc par unité de surface dans la solution aqueuse d'acide acétique de qualité garantie à 8,3% en volume dans l'unité de temps ;
*ρ* et p satisfaisant la relation suivante :
0,1≤15p+ρ≤3,5, p étant supérieur ou égal à 0,005 et inférieur ou égal à 0,14.

2. Dispositif implantable chargé de médicament selon la revendication 1, dans lequel la matrice contenant du zinc est une matrice formée par du zinc ou un alliage de zinc ; ou la matrice contenant du zinc comprend un corps et des zones contenant du zinc attachées au corps ; et
un matériau des zones contenant du zinc est du zinc pur ou un alliage de zinc.

3. Dispositif implantable chargé de médicaments selon la revendication 2, dans lequel l'alliage de zinc est un alliage formé par du zinc et du fer et/ou du magnésium et/ou du palladium et/ou du rhénium et/ou de l'or et/ou de la platine et/ou du tantale et/ou du strontium ; ou l'alliage de zinc est un alliage formé par dopage de zinc avec du carbone et/ou de l'azote et/ou de l'oxygène et/ou du soufre et/ou du bore et/ou du silicium.

4. Dispositif implantable chargé de médicaments selon la revendication 2, dans lequel lorsque la matrice contenant du zinc comprend le corps et les zones contenant du zinc attachées au corps et le matériau des zones contenant du zinc est le zinc pur ou l'alliage de zinc, les zones contenant du zinc recouvrent au moins partiellement la surface du corps, ou les zones contenant du zinc remplissent l'intérieur du corps.

5. Dispositif implantable chargé de médicaments selon la revendication 2, dans lequel lorsque la matrice contenant du zinc comprend le corps et les zones contenant du zinc attachées au corps et le matériau des zones contenant du zinc est l'alliage de zinc, le corps est un corps métallique et les zones contenant du zinc sont formées par réaction entre le corps métallique et le zinc pur.

6. Dispositif implantable chargé de médicaments selon la revendication 2, dans lequel lorsque la matrice contenant du zinc comprend le corps et les zones contenant du zinc attachées au corps et le matériau des zones contenant du zinc est le zinc pur ou l'alliage de zinc, la couche de médicament actif ne recouvre pas du tout les zones contenant du zinc ou la couche de médicament actif recouvre au moins partiellement les zones contenant du zinc.

7. Dispositif implantable chargé de médicaments selon la revendication 1, dans lequel la couche de médicament actif comprend un médicament actif et un support, et le médicament actif est dispersé dans le support.

8. Dispositif implantable chargé de médicaments selon la revendication 7, dans lequel le médicament actif est l'héparine et/ou la colchicine et/ou la rapamycine et/ou un dérivé de celles-ci, et/ou le paclitaxel et/ou un dérivé de celui-ci, et/ou l'hirudine et/ou un dérivé de celle-ci.

9. Dispositif implantable chargé de médicaments selon la revendication 7, dans lequel le support est un polymère dégradable et/ou un polymère non-dégradable et/ou un copolymère formé par au moins deux monomères formant le polymère dégradable, et de monomères formant le polymère non-dégradable ; et dans lequel le polymère dégradable est de l'acide polylactique et/ou de l'acide polyglycolique et/ou de la polycaprolactone et/ou du polysuccinate et/ou du poly(β-hydroxybutyrate), et le polymère non-dégradable est du polystyrène et/ou du polytétrafluoroéthylène et/ou du polyméthacrylate de méthyle et/ou du polycarbonate et/ou du polyéthylène téréphtalate.

10. Dispositif implantable chargé de médicaments selon la revendication 2, dans lequel le corps est réalisé en un métal et le métal est un métal dégradable ou un métal non-dégradable.

11. Dispositif implantable chargé de médicaments selon la revendication 10, dans lequel le métal dégradable est du fer pur, un alliage de fer, du magnésium pur ou un alliage de magnésium.

12. Dispositif implantable chargé de médicaments selon la revendication 10, dans lequel le métal non-dégradable est de l'acier inoxydable 316L, un alliage de de cobalt et de chrome, un alliage de platine et de chrome, du titane pur ou un alliage de titane.

13. Dispositif implantable chargé de médicaments selon la revendication 2, dans lequel le corps est au moins partiellement réalisé en un polymère ; le polymère étant un polymère dégradable et/ou un polymère non-dégradable et/ou un copolymère formé par au moins deux monomères formant le polymère dégradable, et de monomères formant le polymère non-dégradable ; et dans lequel le polymère dégradable est de l'acide polylactique et/ou de l'acide polyglycolique et/ou de la polycaprolactone et/ou du polysuccinate et/ou du poly(β-hydroxybutyrate), et le polymère non-dégradable est du polystyrène et/ou du polytétrafluoroéthylène et/ou du polyméthacrylate de méthyle et/ou du polycarbonate et/ou du polyéthylène téréphtalate.

14. Dispositif implantable chargé de médicaments selon la revendication 1, le dispositif implantable chargé de médicaments étant un tuteur vasculaire, un tuteur biliaire, un tuteur oesophagien, un tuteur urétral, un tuteur pour les voies respiratoires, un implant andrologique, un implant gynécologique ou un filtre de veine cave.
